# EUROPEAN PATENT APPLICATION

(11) **EP 3 275 365 A1**
(43) Date of publication of application: **31.01.2018**
(21) Application number: 16020279.2
(22) Date of filing: 27.07.2016
(51) Int. Cl.: A61B 5/021, A61B 5/0265, A61B 5/05

(54) **NON-INVASIVE INTERACTIVE ARTERIAL CIRCULATION EVALUATION SYSTEM**

(71) Applicant: Cerasani, Ennio, 06800 Cagnes-sur-Mer (FR)
(72) Inventor: Cerasani, Umberto, 06800 CAGNES-SUR-MER (FR)

(57) **Abstract**

A new methodology to continuously, directly and non-invasively measure blood pressure either based on Ultrasound emitter (or Force Sensor). Blood flow is monitored by detection of variation of the Magnetic Field while artery is temporarily subjected to Ultrasound Waves opposing the blood flow (Ultrasound waves can be replaced by placing the Force Sensor on the artery with a manually variable pressure).

This new sensing methodology is based on a "direct" measure of pressure eliminating the need of the pressure servo loop. It can be applied to several arteries such as the Brachial and Radial arteries. It can also be easily extended to allow sensing of echo Ultrasounds for determination of blood flow and artery diameter using Doppler effect and then providing a consistent picture of blood circulation in the artery i.e. Pressure, Blood Flow, artery diameter and macroscopic evaluation of Blood Viscosity.

## Description

Introduction :
Continuous noninvasive arterial blood pressure measurement (CNAP) also called Vascular Unloading Technic is based on measurement of finger blood volume changes using light. The main drawbacks of this method are:
   - Not a direct measure of the pressure (artery diameter is measured)
   - Limited to finger artery (cannot be easily transposed to other arteries)
   - Requires a pressure servo loop

Even if MMSB (Modulated Magnetic Signature of Blood) has been proposed to also be used for blood pressure measurement, it suffers of the lack of linearity and reproducibility.

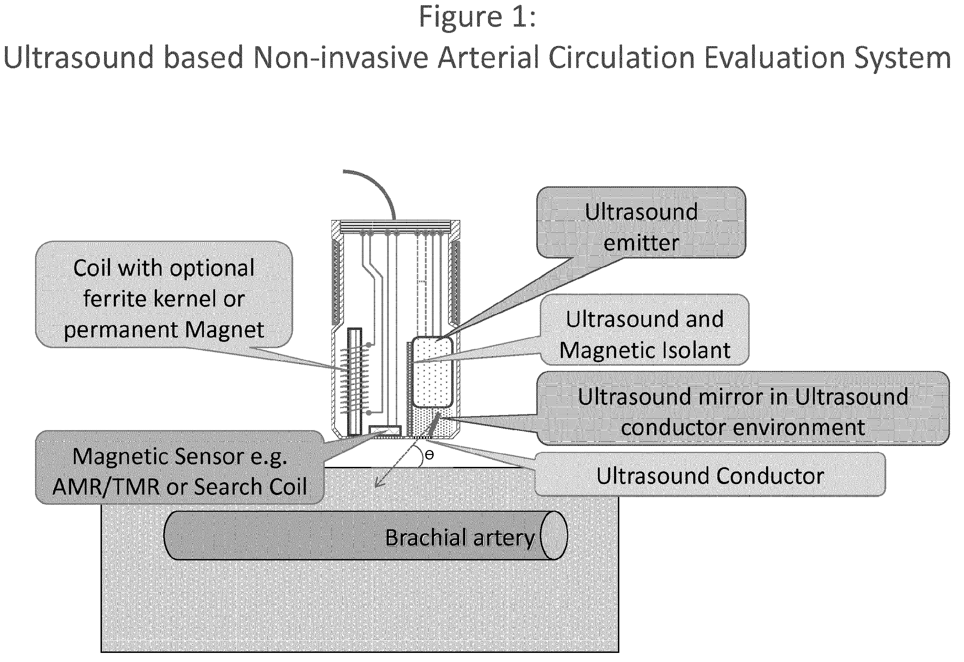

Description :
The system can be split into 5 main parts (also see joined figure 1) :
   - A permanent Magnet or a Coil to slightly magnetize the blood
   - A magnetic sensor such as a Giant Magneto-Resistor (GMR) or a search Coil used in a similar way than on MMSB
   - An Ultrasound Emitter generating variable intensity low frequency Ultrasounds (from 1 to 3 MHz or lower). The Ultrasound emitter can be replaced by a Force sensor
   - An Ultrasound mirror to have the ultrasound waves almost aligned horizontally
   - An Electronic circuit used to control all elements and provide a visual output

Replacing the Ultrasound emitter by an array Ultrasound Transducer (or equivalent) such as a CMUT (Capacitive Micro-machined Ultrasonic Transducer) will allow determination of the artery diameter and Blood flow velocity using Doppler effect.

(Ultrasound Emitter can be replaced by a Force Sensor with variable pressure applied manually.)

## Claims

1. A new methodology to continuously, directly and non-invasively measure blood pressure either based on Ultrasound emitter (or Force Sensor). Analyzed artery is magnetized by a strong, constant and harmless Magnetic Field. Blood flow is monitored by detection of variation of the Magnetic Field using a Search Coil or Anisotropic Magneto-Resistance (AMR) while artery is temporarily subjected to Ultrasound Waves opposing the blood flow (Ultrasound waves can be replaced by placing the Force Sensor on the artery with a manually variable pressure).
This new sensing methodology is based on a "direct" measure of pressure eliminating the need of the pressure servo loop. It can be applied to several arteries such as the Brachial and Radial arteries. It can also be easily extended to allow sensing of echo Ultrasounds for determination of blood flow and artery diameter using Doppler effect and then providing a consistent picture of blood circulation in the artery i.e. Pressure, Blood Flow, artery diameter and macroscopic evaluation of Blood Viscosity.

## Amended claims

### Amended claims in accordance with Rule 137(2) EPC.

1. A new methodology to continuously, directly and non-invasively measure blood pressure typically based on an Ultrasound emitter combined with magnetic detection of blood flow. Analyzed artery is magnetized by a strong, constant and harmless Magnetic Field. Blood flow is monitored by detection of the variation of the Magnetic Field using a Search Coil or Anisotropic Magneto-Resistance (AMR) while artery is temporarily subjected to Ultrasound Waves opposing the blood flow (Ultrasound waves influence can be replaced by a manually operated Force Sensor positioned above the artery).
This new sensing methodology is based on a "direct" measure of blood pressure eliminating the use of a pressure servo loop and/or of a cuff.
It can also be easily extended to allow sensing of echo Ultrasounds for determination of blood flow and artery diameter using Doppler effect and then providing a consistent picture of blood circulation in the artery i.e. Pressure, Blood Flow, artery diameter and macroscopic evaluation of Blood Viscosity.
